# EUROPÄISCHE PATENTSCHRIFT

(11) **EP 3 371 248 B1**
(45) Veröffentlichungstag und Bekanntmachung des Hinweises auf die Patenterteilung: **26.03.2025**
(21) Anmeldenummer: 16787873.5
(22) Anmeldetag: 27.10.2016
(51) Int. Cl.: C08G 77/60

(54) **KONTINUIERLICHES VERFAHREN FÜR REAKTIONEN MIT FEINTEILIGEN ALKALIMETALL-DISPERSIONEN**
CONTINUOUS METHOD FOR REACTIONS WITH FINE-PARTICULATE ALKALI METAL DISPERSIONS
PROCÉDÉ CONTINU POUR RÉALISER DES RÉACTIONS AVEC DES DISPERSIONS DE MÉTAUX ALCALINS À FINES PARTICULES

(30) Priorität: 03.11.2015 DE 102015221529
(43) Veröffentlichungstag der Anmeldung: 12.09.2018
(73) Patentinhaber: CHT Germany GmbH, 72072 Tübingen (DE)
(72) Erfinder: HEES, Michael, 72469 Meßstetten (DE); GEORGI, Ulrike, 72076 Tübingen (DE); BACHUS, Herbert, 72379 Hechingen (DE); MÜLLER, Kai-Sven, 28832 Achim (DE)
(74) Vertreter: dompatent von Kreisler Selting Werner - Partnerschaft von Patent- und Rechtsanwälten mbB
(86) Internationale Anmeldenummer: PCT/EP2016/075956
(87) Internationale Veröffentlichungsnummer: WO 2017/076744

(56) Entgegenhaltungen:
- WO-A1-2006/084878
- DE-B- 1 281 684
- DE-T2- 69 600 862
- GB-A- 896 301
- US-A- 2 631 175

## Beschreibung

Die vorliegende Erfindung betrifft ein kontinuierliches Verfahren, bei dem auf einem Drehscheibenreaktor (engl.: Spinning Disc Reactor (SDR)) feine Alkalimetalldispersionen in organischen Lösungsmitteln generiert und zur Kupplung organischer Ester und Halogenide sowie von Organohalogensilanen eingesetzt werden.

Alkalimetalle, und unter ihnen vor allem Natrium, sind in der organischen Chemie schon lange als Reagenzien etabliert, beispielsweise zur Herstellung von Alkoholaten, für die Glycidesterkondensation nach Darzens, für die Birch-Reduktion zur Umwandlung von aromatischen in aliphatische Verbindungen oder zur Acyloinkondensation von Estern zu α-Hydroxycarbonylverbindungen (K. Rühlmann Synthesis (1971), (1971(5), 236-253). Vor allem letztere Reaktion ist in der organischen Chemie zur intramolekularen Zyklisierung von Dicarbonsäureestern zu mittleren und großen Ringsystemen von Bedeutung.

Ein weiteres großes Anwendungsfeld für elementare Alkalimetalle bietet die 1854 vom französischen Chemiker Adolphe Wurtz entdeckte Wurtz-Kupplung oder Wurtz'sche Synthese, welche anfangs v.a. der Synthese von (Cyclo-)Alkanen ausgehend von Halogenalkanen diente. Mittels der Wurtz'schen-Synthese sind Cycloalkane der Ringgröße von drei bis sechs Kohlenstoffatomen zugänglich. Die Wurtz-Kupplung ist ebenfalls die Haupt-Route zur Herstellung σ-π-konjugierter Siliziumpolymere über die Polykondensation von Halogensilanen in aprotischen organischen Lösungsmitteln (R. D. Miller, J. Michl Chemical Reviews (1989), 89 (6), 1359-1410).

Die genannten Reaktionen werden nach dem heutigen Stand der Technik im Semibatch-Verfahren durchgeführt (unter anderem beschrieben in den Patenten EP 0 123 934 B1, EP 0 632 086 B1, EP 0 382 651 A1, EP 1 769 019 A1, DE 10 2012 223 260 A1, EP 2 872 547 A1, EP 0 230 499 B1, JP 3087921 B2, JP 2736916 B2, CN 103508869 B, US 4324901 A, US 4276424 A). Hierfür wird in einem Reaktionsgefäß die Suspension eines Alkalimetalls (beispielsweise Natrium) vorgelegt. Zu dieser werden unter streng kontrollierten Bedingungen die zu kuppelnden Substanzen (Halogenalkane, Halogensilane oder Di-/Ester) zu dosiert. Dieses Verfahren birgt allerdings eine Vielzahl sicherheitstechnischer Risiken und verfahrenstechnischer Probleme. Zum einen ist der Umgang mit den flüssigen Alkalimetallen, aber auch mit einigen der Reaktanden wie zum Beispiel den hochreaktiven Chlorsilanen im Produktionsmaßstab sehr komplex. So muss in jedem Fall Feuchtigkeit komplett ausgeschlossen werden. Da die Reaktion stark exotherm ist, wird zum anderen die Reproduzierbarkeit der Produkteigenschaften schon durch geringe Schwankungen innerhalb verschiedener Batchansätze beeinflusst. Schon im Labormaßstab wird zum Beispiel eine schlechte Reproduzierbarkeit der Ergebnisse einer Wurtz-Kupplung zur Herstellung von Polysilanen beobachtet.

Da es sich bei Reaktionen mit Alkalimetalldispersionen um Grenzflächenreaktionen handelt, sind eine möglichst feinteilige Dispersion und damit eine hohe Partikeloberfläche vorteilhaft für schnelle und vollständige Reaktionen. Für ein Semibatch-Verfahren ist dies in der Produktion aber nur schwer exakt einstellbar, was zu Abweichungen im Prozess führt. Auch eine exakte Zudosierung der Reaktanden (beispielsweise Halogenverbindungen, Ester) ist für eine exakte Reaktionsführung essentiell, da unterschiedliche Dosierraten zu Abweichungen im Temperaturverlauf führen können. Aus den genannten Gründen ist leicht verständlich, dass diese Faktoren auch das Upscaling des Semibatch-Verfahrens und die damit verbundene großtechnische Nutzung der Reaktion erheblich erschweren.

Für die Reaktion von Alkalimetallen mit Alkoholen über dem Schmelzpunkt der Metalle wurde in EP 1162187 B1 ein Mikroreaktor mit statischem Mischer und Wärmetauscher mit Kanal-Dimensionen um 500 µm verwendet.

Die Verwendung von SDRs ist Stand der Technik für die Epoxidierung von substituierten Cyclohexanonen EP 1 20 6460 B1, Umsetzungen von Carboxysäuren und Estern WO 2002/018328 A1, die Herstellung von Nanopartikeln US 8,870,998 B2, zur Hydrogenierung von Nitrilkautschuk in Lösung EP 1 862 477 B1, für heterogen katalysierte Reaktionen EP 1 152 823 B2, sowie zur Durchführung von radikalischen Emulsionspolymerisationen US 7,683,142 B2.

Darüber hinaus sind in der wissenschaftlichen Literatur Anwendungen für die Herstellung von Titandioxid-Partikeln (S. Mohammadi; A. Harvey; K. V. K. Boodhoo Chemical Engineering Journal (2014), 258, 171-184), zur kationischen Polymerisation von Styrol mithilfe eines immobilisierten Katalysators (K. V. K. Boodhoo; W.A.E. Dunk; M. Vicevic; R. J. Jachuck; V. Sage; D. J. Macquarrie; J. H. Clark Journal of Applied Polymer Science (2006), 101(1), 8-19), zur Photopolymerisation von *n-*Butylacrylat (K. V. K. Boodhoo; W.A.E. Dunk; R. J. Jachuck ACS Symposium Series (2003), 847 (Photoinitiated Polymerization), 437-450), sowie zur Herstellung von Mayonnaise (M. Akhtar; B. S. Murray; S. Dowu Food Hydrocolloids (2014), 42(S), 223-228), Apfelsaftkonzentrat (M. Akhtar; P. Chan; N. Safriani; B. Murray; G. Clayton Journal of Food Processing & Technology (2011), 2(2), 1000108) und Eiscreme-Basisemulsionen (M. Akhtar; I. Blakemore; G. Clayton; S. Knapper International Journal of Food Science and Technology (2009), 44(6), 1139-1145) beschrieben. Weiterhin hat die Firma Flowid einen Lithium-Halogen-Austausch bei Raumtemperatur mit einem Produktfluss von 100 kg/h Rohmaterial beziehungsweise 20kg/h Produkt für Biogen realisiert (http://www.flowid.nl/successful-scale-up-of-butyl-lithium-usingthe-spinpro/ 08.08.2015). Außerdem sind in der Literatur viele Beispiele zu Rekristallisation beziehungsweise Partikelsynthese beschrieben. Die Vorteile der SDR-Technologie für diese mehrphasigen Systeme wurden in der Fachliteratur ausführlich besprochen und mit Beispielen belegt (K. Boodhoo & A. Harvey Process Intensification for Green Chemistry: Engineering Solutions for Sustainable Chemical Processing (2013), Chapter 3, John Whiley & Sons Ltd, pp 59-90), S. D. Pask, O. Nuyken, Z. Cai Polymer Chemistry (2012), 3, 2698), P. Oxley, C. Brechtelsbauer, F. Ricard, N. Lewis, C. Ramshaw Industrial & Engineering Chemistry Research (2000), 39(7), 2175-2182).

DE 696 00 862 T2 offenbart ein sicheres, effizientes Verfahren zur Herstellung von Natrium-C4-C8-Alkoxid, das eine weniger als stöchiometrische Menge eines C4-C8-Alkohols und die Möglichkeit einer kontinuierlichen Rückführung des nicht umgesetzten Natriummetalls verwendet.

US 2,631,175 A beschreibt ein Verfahren zur Herstellung von Polybutadien. Insbesondere wird ein kontinuierliches Verfahren offenbart, in welchem eine feinteilige Natriumdispersion als Katalysator eingesetzt wird.

WO 2006/084878 A1 offenbart Verfahren zur Herstellung tertiärer Phosphine durch Umsetzung einer Verbindung der allgemeinen Formel (I) A,B-P-L1, in der A für R1 oder L2, B für R2 oder L3, die Reste R1 und R2 unabhängig voneinander für einen organischen Rest mit jeweils 1 bis 30 Kohlenstoffatomen, wobei die Reste R1 und R2 auch miteinander verbunden sein können; und die Abgangsgruppen L1 bis L3 unabhängig voneinander für Halogen, Alkyloxy mit 1 bis 10 Kohlenstoffatomen oder Aryloxy mit 6 bis 10 Kohlenstoffatomen stehen, (a) mit einem Alkalimetall in einem organischen aprotischen Lösungsmittel und (b) einer Verbindung der allgemeinen Formel (II) L4-R3 in der der Rest R3 für einen organischen Rest mit jeweils 1 bis 30 Kohlenstoffatomen; und die Abgangsgruppe L4 für Halogen, Alkyloxy mit 1 bis 10 Kohlenstoffatomen oder Aryloxy mit 6 bis 10 Kohlenstoffatomen, bei dem man das erhaltene Reaktionsgemisch mit einer wässrigen Base, welche einen pH-Wert von >= 10 aufweist, hydrolysiert und die organische Phase von der wässrigen Phase abtrennt.

Der vorliegenden Erfindung liegt nun die Aufgabe zugrunde, ein kontinuierliches Verfahren zur Durchführung von chemischen Reaktionen mit flüssigen feinteiligen Alkalimetall-Dispersionen in inerten Lösungsmitteln zur Verfügung zu stellen, welches nicht durch die beschriebenen Probleme der Semibatch-Fahrweise limitiert ist.

Überraschenderweise wurde gefunden, dass die vorgenannte Aufgabe in einer ersten Ausführungsform gelöst wird durch die Verwendung einer Vorrichtung zur Durchführung kontinuierlicher chemischer Reaktionen zur Umsetzung von feinteiligen Alkalimetall-Dispersionen in inerten Lösungsmitteln, beziehungsweise erfindungsgemäß durch die Verwendung eines Drehscheibenreaktors zur Durchführung von kontinuierlichen chemischen Verfahren unter Einsatz von flüssigen feinteiligen Dispersionen von Alkalimetallen, Alkalimetallgemischen und/ oder Alkalimetalllegierungen in inerten Lösungsmitteln.

Bekannte Systeme der kontinuierlichen Produktionstechnik sind sogenannte Strömungsrohrreaktoren (engl. Plug Flow Reactors, PFRs), kontinuierliche Rührkessel (engl. Continuously Stirred Tank Reactor, CSTR) und deren Kaskaden, sowie die Mikroreaktionstechnik (MRT). Für die normalerweise stark exothermen Grenzflächenreaktionen an dispergierten Alkalimetallen ist eine hocheffiziente Durchmischung essentiell, um einen möglichst hohen Massefluss der Reaktanden und Produkte an die beziehungsweise von der Oberfläche zu gewährleisten, sowie die entstehende Wärme schnell abzuführen. Dies kann zum Beispiel mit statischen Mixern in Strömungsrohr- beziehungsweise Mikroreaktoren erreicht werden. Diese weisen weiterhin eine ideale Verweilzeitverteilung auf. Auch in CSTRs ist eine gute Durchmischung möglich; sie haben den zusätzlichen Vorteil, dass sie vergleichsweise leicht zu reinigen sind, wodurch Fouling beziehungsweise Blocking bei mehrphasigen Reaktionen (z.B. mit zwei nicht mischbaren flüssigen Komponenten oder festen Reaktionsprodukten) ein geringes Problem darstellt.

Ein bevorzugtes bisher technisch wenig eingesetztes System der kontinuierlichen Produktionstechnik ist der Drehscheibenreaktor (*engl.* Spinning Disc Reactor, SDR).

Erfindungsgemäß können die wesentlichen Probleme eines Semibatchverfahrens für die Durchführung von Reaktionen mit feinteiligen Alkalimetalldispersionen durch eine kontinuierliche Fahrweise beseitigt werden: Bei einem kontinuierlichen Reaktionsprozess werden nur kleine Mengen von Gefahrstoffen pro Zeiteinheit zusammengeführt und hierbei idealerweise effizient durchmischt. Wird eine stark exotherme Reaktion gehandhabt, kann die entstehende Wärme durch ein hohes Oberfläche-Volumen-Verhältnis gut abgeführt werden und es resultiert ein homogenes Temperaturprofil in der Reaktionsmischung. Dies alles führt erfindungsgemäß zu einer signifikanten Verbesserung des Sicherheitsaspekts solcher Reaktionen, sowie zu einer deutlich verbesserten Reproduzierbarkeit der Produktqualität im stationären Zustand eines solchen kontinuierlichen Prozesses.

Die vorliegende Erfindung findet insbesondere Anwendung für die Wurtz-Kupplung von Halogenverbindungen, sowie für die Acyloinkondensation von Estern. Das hierbei jeweils vorliegende flüssige Zweiphasensystem vor der Reaktion bzw. die bei der Wurtz-Kupplung entstehende konzentrierte Salzdispersion führt im kontinuierlich betriebenen SDR aufgrund der hohen Scherkräfte in diesem dynamischen System nicht zu Fouling und Verblockungen.

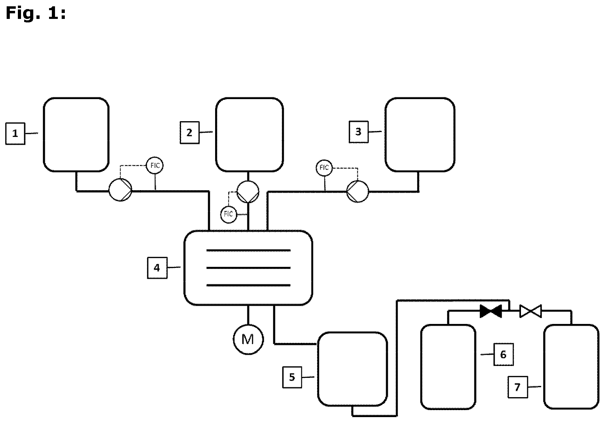

Fig. 1 zeigt ein exemplarisches Fließschema für die vorliegende Erfindung: Das Bezugszeichen 1 steht für einen Dosierbehälter mit flüssigem Alkalimetall beziehungsweise Alkalimetallmischung/-legierung; Das Bezugszeichen 2 steht für einen Dosierbehälter des Reaktanden beziehungsweise eine Reaktandenmischung; Das Bezugszeichen 3 steht für einen Dosierbehälter des Lösungsmittels beziehungsweise eine Lösungsmittelmischung; Das Bezugszeichen 4 steht für einen Drehscheibenreaktor (Spinning Disc Reaktor, SDR); Das Bezugszeichen 5 steht für eine optionale Verweilzeiteinheit (beispielsweise Rührkessel, Rührkesselkaskade, Wärmetauscher); Das Bezugszeichen 6 steht für einen Auffangbehälter der Produktmischung; Das Bezugszeichen 7 steht für einen Auffangbehälter der Nebenproduktmischung.

Aufgrund der oben genannten Beispiele konnte erfindungsgemäß der Drehscheibenreaktor für die Durchführung von Reaktionen mit flüssigen Alkalimetalldispersionen in inerten organischen Lösungsmitteln, insbesondere der Wurtz-Kupplung von Halogenverbindungen sowie der Acyloinkondensation, unter Bildung fester Niederschläge als geeignete Technologie entwickelt werden. Dieser Reaktortypus wurde für die Prozessintensivierung entwickelt um besonders Wärmetransport (Heizen, Kühlen, Wärmeaustausch) und Stofftransportprozesse (Mischen, Dispergieren) zu optimieren: Die Technologie basiert auf einer oder mehreren Scheiben, die horizontal auf einer rotierenden Achse angebracht sind, welche von einem elektrischen Motor angetrieben wird. Eine auf die Oberfläche dieser Scheibe aufgebrachte Flüssigkeit wird durch die wirkende Zentrifugalbeschleunigung an den Rand der Platte beziehungsweise darüber hinausgeschleudert. In dem gebildeten dünnen Film wirken hohe Scherkräfte und damit ausgezeichnete Wärme- und Massentransferraten. So kann bei stark exothermen Reaktionen die entstehende Wärme schnell abgeführt werden, sowie bei diffusionslimitierten Prozessen der Stofftransport maximiert werden. Die hohen in den durchfließenden Flüssigkeitsfilm eingebrachten Scherkräfte führen außerdem zu starken Verwirbelungen, was vor allem bei Reaktionen in mehrphasigen Systemen entscheidende Vorteile bietet: Durch die hohen Scherkräfte wird die dispergierte Phase sehr fein verteilt und die Grenzfläche zwischen zwei nicht mischbaren Phasen maximiert. Dadurch wird der Massentransport zur Grenzfläche hin und von dieser fort optimiert. Weiterhin ist die Rotationsbewegung stark genug, um feste Ablagerungen von der Scheibenoberfläche zu schleudern, wodurch eine Art Selbstreinigungsmechanismus eintritt und Fouling beziehungsweise Blocking durch die Bildung von Niederschlag reduziert wird.

Typischerweise findet die in dieser Erfindung beschriebene Verwendung in einem flüssigen Medium statt, welches aus einem inerten Lösungsmittel beziehungsweise Lösungsmittelgemisch sowie den darin gelösten Reaktanden (beispielsweise Halogenalkane, Halogensilane, Ester) besteht. Im Allgemeinen ist es zum Erreichen einer hohen Reaktionsgeschwindigkeit notwendig, dass das verwendete Alkalimetall beziehungsweise die verwendete Alkalimetallmischung oder-legierung in geschmolzenem Zustand vorliegt, um es in dem Reaktionsmedium feinteilig zu dispergieren. Das Alkalimetall beziehungsweise die Alkalimetallmischung oder -legierung, die Reaktanden und Lösungsmittel werden jeweils separat in den Reaktor dosiert. Bei der Reaktion des Alkalimetalls beziehungsweise der Alkalimetallmischung beziehungsweise -legierung mit den Reaktanden entsteht ein im Reaktionsgemisch unlösliches Alkalimetallsalz, sowie je nach Reaktionstypus ein im flüssigen Medium lösliches oder auch unlösliches Kupplungsprodukt. Es handelt sich damit um eine kontinuierlich durchgeführte Mehrphasenreaktion, bei der die reaktiven Phasen flüssig vorliegen und ein oder mehrere Reaktionsprodukte als fester Niederschlag ausfallen.

Alle Reaktionsschritte finden typischerweise unter Inertgasatmosphäre statt, um Nebenreaktionen durch Sauerstoff oder Luftfeuchtigkeit, insbesondere die Bildung von Wasserstoff und Chlorwasserstoff, zu verhindern.

Zur Herstellung der Alkalimetalldispersion sind prinzipiell alle Alkalimetalle, deren Mischungen und/oder Legierungen geeignet. Bevorzugt sind Alkalimetalle beziehungsweise Mischungen und/oder Legierungen dieser, deren Schmelzpunkt im Bereich zwischen -20 und 190°C liegt. Besonders bevorzugt sind Lithium, Natrium und Kalium sowie deren Mischungen und Legierungen, da sie technisch gut zugänglich sind und in einem Temperaturbereich schmelzen, der technisch gut handhabbar ist.

Als inerte Lösungsmittel können alle technisch gängigen aprotischen organischen Lösungsmittel eingesetzt werden, in denen die Reaktanden (beispielsweise Halogenverbindungen der Elemente der IV. Hauptgruppe beziehungsweise organische Ester) löslich sind und die nicht mit dem eingesetzten Alkalimetall beziehungsweise der eingesetzten Alkalimetallmischung oder -legierung reagieren. Zum Einsatz kommen bevorzugt Kohlenwasserstoffe wie Benzol, Toluol, Xylol und aliphatische Kohlenwasserstoffe, sowie Ether wie Dioxan, Anisol und THF. Das inerte Lösungsmittel kann ebenfalls eine Mischung der genannten Lösungsmittel sein, z. B. aus einem Kohlenwasserstoff und einem Ether, also beispielsweise Toluol und THF. Typischerweise ist das in der Reaktion gebildete Alkalimetallsalz in dem verwendeten Lösungsmittel nicht löslich und kann durch Filtration des Niederschlages abgetrennt werden.

Als Reaktanden kommen verschiedene Substanzen in Frage:
Mit dem erfindungsgemäßen Verfahren können alle für die Wurtz-Kupplung gängigen Substrate wie mono-, di-, tri- und polyfunktionelle Alkyl- und Aryl-Halogenide und deren Mischungen sowie mono-, di-, tri- und polyfunktionelle Halogenorganosilane und deren Mischungen umgesetzt werden. Als Halogene in den Halogenverbindungen können Fluor, Chlor, Brom und Iod zum Einsatz kommen, bevorzugt kommen Chlor, Brom und Iod, besonders bevorzugt Chlor und Brom zum Einsatz. Insbesondere ist die Erfindung zur Herstellung von Polysilanen und Polycarbosilanen aus halogenierten Organosilan-Bausteinen, die keine Polysilane sind, geeignet. Die halogenierten Organosilan-Bausteine sind definiert dadurch, dass sie weniger als 5 Siliziumatome, bevorzugt maximal 3 Siliziumatome, besonders bevorzugt maximal 2 Siliziumatome und ganz besonders bevorzugt lediglich ein Siliziumatom aufweisen.

Für die Acyloinkondensation kommen Monoester, Diester, Triester und Poly-Ester zum Einsatz. Bevorzugt kommen Mono- und Diester zum Einsatz. Insbesondere ist das beschriebene Verfahren geeignet zur intramolekularen Zyklisierung von Dicarbonsäureestern zu mittleren und großen Ringsystemen.

Bei der vorliegenden Erfindung ist die Menge des Lösungsmittels, des Alkalimetalls und der Reaktanden so zu wählen, dass das Reaktionsmedium auch nach Bildung der fest ausfallenden Alkalimetallhalogenide und ggf. der unlöslichen Produkte fließfähig bleibt, damit Fouling und Verblockungen des SDR minimiert bleiben. Typischerweise werden 5 bis 99 Gew. % Lösungsmittel bezogen auf die Gesamtreaktionsmischung eingesetzt, bevorzugt 50 bis 97 Gew. %, besonders bevorzugt sind 70 bis 95 Gew. % Lösungsmittelanteil.

Erfindungsgemäß wird das Verhältnis der eingesetzten Alkalimetalle beziehungsweise deren Mischungen und/oder Legierungen zu den Reaktanden so gewählt, dass ein möglichst vollständiger Umsatz erzielt wird. Bei der Herstellung von niedermolekularen Kopplungsprodukten ist ein möglichst stöchiometrischer Einsatz bevorzugt. Für die Herstellung von polymeren Kupplungsprodukten benötigt man typischerweise einen Überschuss des Alkalimetalls gegenüber dem Halogenid, bevorzugt einen Überschuss von bis zu 20 Gew. %, besonders bevorzugt einen Überschuss von bis zu 10 Gew. %, ganz besonders bevorzugt einen Überschuss von bis zu 5 Gew. %.

Der in dieser Erfindung beschriebene Prozess kann in einem weiten Temperaturbereich durchgeführt werden. Der Temperaturbereich ist dadurch limitiert, dass es sich um einen mehrphasigen Prozess mit zwei Flüssigkeiten, genauer dem geschmolzenen Alkalimetall beziehungsweise der geschmolzenen Alkalimetallmischung beziehungsweise -legierung und dem Reaktionsmedium aus Lösungsmittel und Reaktanden, handelt. Damit ergibt sich als untere Grenze für den Temperaturbereich die Schmelztemperatur des Alkalimetalls beziehungsweise der Alkalimetalllegierung/-mischung. Die obere Grenze ergibt sich aus dem Siedebereich des Reaktionsgemisches und der technischen Auslegung des SDRs. Bevorzugt wird die Reaktion in einem Bereich zwischen 50 und 200 °C ausgeführt, besonders bevorzugt zwischen 100 und 115°C.

Die Rotationsgeschwindigkeit der Drehscheibe des SDR muss so gewählt werden, dass eine optimale Dispersion des flüssigen Alkalimetalls beziehungsweise der Alkalimetallmischung /-legierung im Lösungsmittel erzielt wird.

Ein weiterer Vorteil der vorliegenden Erfindung ist das hohe Verhältnis von Oberfläche zu Volumen der Reaktionsmischung und die effiziente Durchmischung dieser, wodurch ein effizienter Abtransport der in der exothermen Reaktion generierten Wärmemenge möglich ist. Daraus resultieren eine kurze Verweilzeit der Reaktionsmischung im SDR und damit eine kurze Gesamtprozessdauer bei gleichzeitiger Verminderung des Gefahrenpotentials der Reaktion. Die daraus resultierende hohe Raum-Zeit-Ausbeute macht das beschriebene Verfahren im Vergleich zu etablierten Semibatch-Verfahren wirtschaftlich höchst interessant. Typischerweise ist die Verweilzeit der Reaktionsmischung im SDR <10 min, besonders bevorzugt <5 min. In dieser Zeit wird der größte Teil der Exothermie der Reaktion freigesetzt und abgeführt. Um speziell bei der Herstellung von polymeren Reaktionsprodukten den Umsatz der reaktiven Gruppen zu maximieren, kann gegebenenfalls eine Verweilzeiteinheit nachgeschaltet werden. Hierbei kommen alle dem Fachmann bekannten geeigneten Einheiten in Frage, insbesondere ein kontinuierlicher Rührkessel, eine Rührkesselkaskade, ein Wärmetauscher und thermostatierbare Schlaufenreaktoren.

Wenn die Reaktion den gewünschten Umsetzungsgrad erreicht hat, kann das Reaktionsprodukt aus der Reaktionsmischung durch jegliche dem Fachmann bekannte geeignete Methode abgetrennt und ggf. aufgereinigt. Ist beispielsweise das Produkt im Reaktionsmedium löslich, wird zuerst der feste Alkalimetallsalz-Niederschlag mit gegebenenfalls restlichem Alkalimetall durch Filtration abgetrennt. Dann wird das Produkt beispielsweise durch fraktionierte Destillation oder Abdestillation des Lösungsmittels erhalten. Ist das Produkt unlöslich im Reaktionsmedium, kann es durch Filtration zusammen mit dem festen Alkalimetallsalz-Niederschlag und ggf. restlichem Alkalimetall durch Filtration abgetrennt werden. Nicht umgesetztes Alkalimetall wird ggf. mit protischen Lösungsmitteln deaktiviert und die Alkalimetallsalze mit Wasser extrahiert. Dies sind Beispiele.

### Ausführungsbeispiele:

Für die durchgeführten Reaktionen wurde ein handelsüblicher SpinPro Reactor der Firma Flowid (Eindhoven, Niederlande, http://www.flowid.nl/spinpro-reactor/) mit drei Drehscheiben und integriertem Wärmetauscher verwendet. Der Abstand der Drehscheiben von der Wandung betrug 2 mm. Das Reaktorvolumen betrug 230 ml. Für alle Reaktionen wurde der Reaktor zur Inertisierung 20 mal evakuiert und mit 8 bar Stickstoff belüftet. Für die Synthese der Polysilane (Beispiele 1 und 2) wurde als Verweilzeiteinheit ein kontinuierlicher Rührkessel mit einer Verweilzeit von 20 min (Fluss 7,2 kg/h => 2,8 L; Fluss 10,8 kg/h => 4,1 L) nach den SDR geschaltet.

### Vergleichsbeispiel:

Semibatch-Verfahren zur Herstellung von Poly(methylphenylsilan):
Unter Verwendung von Standard-Schlenk-Technik wurde eine Apparatur aus einem 1000mL Vierhalskolben mit Tropftrichter, Rückflusskühler, KPG-Rührer und Temperaturfühler evakuiert und unter Argon gesetzt. Im Vierhalskolben wurden 215 g Xylol und 17,9 g Natrium vorgelegt. Der Tropftrichter wurde ebenfalls unter Inertgasatmosphäre mit 67,3 g Dichlormethylphenylsilan befüllt. Unter langsamem Rühren wurde der Kolben im Ölbad auf ca. 102 °C erhitzt, so dass das Natrium geschmolzen vorlag. Nun wurde die Rührgeschwindigkeit auf genau 300 rpm eingestellt und etwa fünf Minuten gerührt. Nach dieser Zeit hatte sich eine homogene, feinteilige Dispersion gebildet (optische Beurteilung). Zu dieser Suspension wurde über einen Zeitraum von etwa 30 min das Dichlorphenylmethylsilan zu dosiert. Das Anspringen der Reaktion konnte über ein Ansteigen der Temperatur, sowie eine intensive Violettfärbung der Reaktionsmischung beobachtet werden. Die Dosiergeschwindigkeit war dabei so zu wählen, dass das Dichlorphenylmethylsilan gleichmäßig über die Dosierzeit hinweg zugegeben wurde und gleichzeitig die Reaktionstemperatur stets im Temperaturfenster von 102 - 106°C blieb.

Die Reaktionsmischung wurde nach Ende der Dosierung noch 2 h bei 102°C nachgerührt. Die gebildete Suspension wurde nach Abkühlen auf Raumtemperatur unter Schutzgas über eine Keramikfritte (G4) filtriert. Das Filtrat wurde durch Abdestillieren des Lösungsmittelgemisches im Vakuum konzentriert, um den löslichen Anteil des gebildeten Po-ly(methylphenylsilans) zu erhalten. Das Polymer wurde mittels ¹H-NMR-¹³C-NMR-, ²⁹Si-NMR-Spektroskopie (jeweils in C₆D₆) und Gelpermeationschromatographie (in THF mit Lichtstreudetektor) charakterisiert.
Ausbeute: 32 %
Molmasse: 2000 g/mol
¹H-NMR (δ [ppm], CDCl₃): 6,5-7,5 ppm, 0,5 ppm.
¹³C-NMR (δ [ppm], CDCl₃): 134-138 ppm, 125-130 ppm, 21 ppm.
²⁹Si-NMR (δ [ppm], CDCl₃): -41 ppm, -40 ppm, -39 ppm, +15 ppm.

### Beispiel 1 - Kontinuierliche Herstellung von Poly(methylphenylsilan):

Zum Anfahren der Reaktion wurde der SDR auf eine Umdrehungsgeschwindigkeit von 1000 oder 2500 rpm, eine Heizöltemperatur von 100 oder 105°C und anfangs einen Xylolfluss von 6,5 kg/h eingestellt. Sobald die sich eine konstante Temperatur im Reaktor eingestellt hatte, wurde die Natrium-Dosierung gestartet. Sobald der Austritt einer grauen Dispersion in den Auffangbehälter beobachtet wurde, wurde die Dosierung des Dichlormethylphenylsilans gestartet. Das Masseverhältnis von Dichlormethylphenylsilan zu Natrium war bei allen Versuchen mit 5/1 konstant. Der Beginn der Reaktion war sofort erkennbar durch den Temperaturanstieg auf der ersten und zweiten Platte des Reaktors. Später trat das tief-violett gefärbte Reaktionsgemisch in den Auffangbehälter aus. Die Dosierraten und Öltemperaturen wurden wie in der Tabelle aufgeführt variiert.

Das Produkt wurde unter Stickstoff über eine G4-FKeramikfitte abfiltriert und das Lösemittel wurde unter Vakuum vollständig abdestilliert. Das Polymer wurde mittels ¹H-NMR-, ¹³C-NMR-, ²⁹Si-NMR-Spektroskopie (jeweils in C₆D₆) und Gelpermeationschromatographie (in THF mit Lichtstreudetektor) charakterisiert.

| | **Drehzahl [rpm]** | **T_{Heizöl} [°C]** | **Gesamtfluss [kg/h]** | **Feststoffgehalt [%]** | **T_{1./2./3.Platte} [°C]** | **Molmasse [g/mol]** | **Ausbeute Isl. Polysilan [%]** |
|---|---|---|---|---|---|---|---|
| 1.1 | *2500* | *105* | *7,2* | *10* | *109*/*107*/*104* | 3100 | 46 |
| 1.2 | -"- | -"- | *10,8* | -"- | *112*/*108*/*105* | 2900 | 43 |
| 1.3 | *2500* | *105* | 10,8 | *20* | 119/114/109 | 1900 | 35 |
| 1.4 | -"- | -"- | 7,2 | -"- | 116/113/108 | 2200 | 38 |
| 1.5 | 1000 | 105 | 7,2 | 20 | 106/111/107 | 2400 | 41 |
| 1.6 | -"- | -"- | 10,8 | -"- | 107/118/109 | 2100 | 37 |
| 1.7 | *2500* | *100* | *10,8* | *20* | *110*/*106*/*101* | 2900 | 42 |
| 1.8 | -"- | -"- | *7,2* | -"- | *107*/*103*/*100* | 3200 | 47 |

| | | | | | | | |
|---|---|---|---|---|---|---|---|
| ¹H-NMR (δ [ppm], CDCl₃): 6,5-7,5 ppm, 0,5 ppm. ¹³C-NMR (δ [ppm], CDCl₃): 134-138 ppm, 125-130 ppm, 21 ppm. ²⁹Si-NMR (δ [ppm], CDCl₃): -41 ppm, -40 ppm, -39 ppm, +15 ppm. | | | | | | | |

### Beispiel 2 - Herstellung von zyklischem Oligo(methylphenylsilan):

Bei einer Umdrehungsgeschwindigkeit von 2500 rpm, einer Heizöltemperatur von 125°C und einem Xylolfluss von 6,5 kg/h wurde die Natrium-Dosierung mit 2 g/min gestartet. Die Temperatur im Reaktor pendelte sich auf 122°C ein. Sobald der Austritt einer grauen Dispersion aus dem Reaktor beobachtet wurde, wurde die Monomerdosierung mit 10 g/min gestartet. Der Beginn der Reaktion war sofort erkennbar durch den Temperaturanstieg im Reaktor auf 129°C auf der ersten Platte. Etwas später trat das tiefviolett gefärbte Reaktionsgemisch in den Auffangbehälter aus. Das Produkt wurde unter Stickstoff abfiltriert und das Lösemittel wurde abdestilliert. Das Polymer wurde mittels ¹H-NMR-, ¹³C-NMR-, ²⁹Si-NMR-Spektroskopie (jeweils in C₆D₆) und Gelpermeationschromatographie (in THF mit Lichtstreudetektor) charakterisiert.
GPC (Elutionsmittel Toluol, RI-Detektor, Kalibrierung: Polysiloxan-Standard): 300 g/mol, 900 g/mol
GPC (Elutionsmittel THF, LS-Detektor, probenspezifische Kalibrierung): 1300 g/mol, Ð=1,6
¹H-NMR (δ [ppm], CDCl₃): 6,5-7,5 ppm, 0,5 ppm.
¹³C-NMR ([δ ppm], CDCl₃): 134-138 ppm, 125-130 ppm, 21 ppm.
²⁹Si-NMR (δ [ppm], CDCl₃): -41 ppm, -40 ppm, -39 ppm.

### Beispiel 3 - Ringschluss von 1,6-Dibromhexan zu Cyclohexan:

Bei einer Umdrehungsgeschwindigkeit von 2500 rpm, einer Heizöltemperatur von 110°C und einem Xylolfluss von 6,5 kg/h wurde die Natrium-Dosierung mit 1,0 g/min gestartet. Die Temperatur im Reaktor pendelte sich auf 122°C ein. Sobald der Austritt einer grauen Dispersion aus dem Reaktor beobachtet wurde, wurde die Dosierung von 1,6-Dibromhexan mit 4 g/min gestartet. Der Beginn der Reaktion war sofort erkennbar durch den Temperaturanstieg im Reaktor auf 127°C auf der ersten Platte. Etwas später trat das tief-violett gefärbte Reaktionsgemisch in den Auffangbehälter aus. Das Produkt wurde unter Stickstoff abfiltriert, die Zusammensetzung des Filtrats mittels GC-MS analysiert und ein Umsatz von 98,5 % bestimmt. Das reine Produkt wurde durch fraktionierte Destillation des Filtrats in 91 % Ausbeute erhalten und mittels ¹H- und ¹³C-NMR-Spektroskopie analysiert.
¹H-NMR (δ [ppm], CDCl₃): 1,44.
¹³C-NMR (δ [ppm], CDCl₃): 26,9.

## Patentansprüche

1. Verwendung einer Vorrichtung zur Durchführung kontinuierlicher chemischer Reaktionen zur Umsetzung von feinteiligen Alkalimetall-Dispersionen in inerten Lösungsmitteln, **dadurch gekennzeichnet, dass** man als Vorrichtung einen Drehscheibenreaktor einsetzt.

2. Verwendung nach Anspruch 1, **dadurch gekennzeichnet, dass** man als Alkalimetall Lithium, Natrium, Kalium, deren Gemische und/oder Legierungen einsetzt.

3. Verwendung nach Anspruch 1 oder 2, **dadurch gekennzeichnet, dass** das chemische Verfahren eine Wurtz'sche Synthese ist.

4. Verwendung nach Anspruch 3, **dadurch gekennzeichnet, dass** man als Reaktanden für die Wurtz'sche Synthese mono-, di- oder multifunktionellen Halogenverbindungen der Elemente der IV. Hauptgruppe einsetzt.

5. Verwendung nach Anspruch 3 oder 4 zur Herstellung von linearen, verzweigten und cyclischen Kohlenwasserstoffen aus mono-, di- oder multifunktionellen organischen Halogenverbindungen und Mischungen dieser bei der Wurtz'sche Synthese.

6. Verwendung nach Anspruch 4 oder 5 zur Herstellung von Polysilanen, insbesondere Polycarbosilanen aus mono-, di- oder multifunktionellen Halogensilanen und Mischungen dieser.

7. Verwendung nach Anspruch 1 oder 2, **dadurch gekennzeichnet, dass** das chemische Verfahren eine Acyloinkondensation ist.

8. Verwendung nach Anspruch 7, **dadurch gekennzeichnet, dass** man als Reaktanden für die Acyloinkondensation organische Mono-, Di-, Tri- oder Poly-Ester-Verbindungen einsetzt.

9. Verwendung nach einem der Ansprüche 1 bis 8, **dadurch gekennzeichnet, dass** der Drehscheibenreaktor eine nachgeschaltete Verweilzeiteinheit aufweist.

## Claims

1. Use of a device for performing continuous chemical reactions for reacting finely disperse alkali metal dispersions in inert solvents, **characterized by** that a rotary disk reactor is employed as said device.

2. The use according to claim 1, **characterized by** that lithium, sodium, potassium, mixtures thereof and/or alloys thereof are employed as said alkali metal.

3. The use according to claim 1 or 2, **characterized by** that the chemical reaction is a Wurtz reaction.

4. The use according to claim 3, **characterized by** that mono-, di- or multifunctional halogen compounds of the elements of group 14 of the Periodic Table are employed as reactants for said Wurtz reaction.

5. The use according to claim 3 or 4 for preparing linear, branched and cyclic hydrocarbons from mono-, di- or multifunctional organic halogen compounds and mixtures thereof in a Wurtz reaction.

6. The use according to claim 4 or 5 for preparing polysilanes, especially polycarbosilanes, from mono-, di- or multifunctional halogen silanes and mixtures thereof.

7. The use according to claim 1 or 2, **characterized by** that the chemical reaction is an acyloin condensation.

8. The use according to claim 7, **characterized by** that organic mono-, di-, tri- or polyester compounds are employed as reactants for said acyloin condensation.

9. The use according to any of claims 1 to 8, **characterized by** that said rotary disk reactor has a downstream dwelling time unit.

## Revendications

1. Utilisation d'un dispositif pour effectuer des réactions chimiques continues pour faire réagir des dispersions de métaux alcalins finement divisés dans des solvants inertes, **caractérisée en ce qu'**un réacteur à disque tournant est utilisé comme ledit dispositif.

2. Utilisation selon la revendication 1, **caractérisée en ce que** le lithium, le sodium, le potassium ou des mélanges de ceux-ci et/ou des alliages de ceux-ci sont utilisés comme métal alcalin.

3. Utilisation selon la revendication 1 ou 2, **caractérisée en ce que** le procédé chimique est une réaction de Wurtz.

4. Utilisation selon la revendication 3, **caractérisée en ce que** des composés halogénés mono-, di- ou multifonctionnels des éléments du groupe 14 du tableau périodique sont utilisés pour la réaction de Wurtz.

5. Utilisation selon la revendication 3 ou 4 pour préparer des hydrocarbures linéaires, ramifiés et cycliques à partir de composés halogénés organiques mono-, di- ou multifonctionnels et des mélanges de ceux-ci dans la réaction de Wurtz.

6. Utilisation selon la revendication 4 ou 5 pour préparer des polysilanes, notamment des polycarbosilanes, à partir d'halosilanes mono-, di- ou multifonctionnels et des mélanges de ceux-ci.

7. Utilisation selon la revendication 1 ou 2, **caractérisée en ce que** le procédé chimique est une condensation acyloïne.

8. Utilisation selon la revendication 7, **caractérisée en ce que** des composés mono-, di-, tri- ou polyesters organiques sont utilisés comme réactants pour la condensation acyloïne.

9. Utilisation selon l'une quelconque des revendications 1 à 8, **caractérisée en ce que** ledit réacteur à disque tournant présente une unité de temps de séjour située en aval.
